# EUROPEAN PATENT SPECIFICATION

(11) **EP 1 343 530 B1**
(45) Date of publication and mention of the grant of the patent: **08.08.2007**
(21) Application number: 01983010.8
(22) Date of filing: 07.11.2001
(51) Int. Cl.: A61K 47/34, A61P 7/02

(54) **ORAL PHARMACEUTICAL COMPOSITION CONTAINING A BLOCK COPOLYMER**
ORALE PHARMAZEUTISCHE ZUSAMMENSETZUNG MIT EINEM BLOCKCOPOLYMER
COMPOSITION PHARMACEUTIQUE ADMINISTREE PAR VOIE ORALE CONTENANT UN COPOLYMERE SEQUENCE

(30) Priority: 09.11.2000 GB 0027375; 27.02.2001 GB 0104751
(43) Date of publication of application: 17.09.2003
(73) Proprietor: AstraZeneca AB, 151 85 Södertälje (SE)
(72) Inventor: BATEMAN, Nicola, AstraZeneca R & D Alderley, Macclesfield, SK10 4TF Cheshire (GB); CAHILL, Julie, AstraZeneca, Macclesfield, SK10 2NA Cheshire (GB)
(86) International application number: PCT/SE2001/002470
(87) International publication number: WO 2002/038184

(56) References cited:
- WO-A1-00/19996
- WO-A1-99/18142
- WO-A1-99/57113
- US-A- 5 665 428

## Description

The invention relates to oral pharmaceutical compositions which comprise a water miscible micelle forming block copolymer (hereinafter called "the copolymer") and a compound. The copolymer can be a diblock copolymer of formula AB or BA. However the copolymer could also be a triblock copolymer of formula ABA or BAB. The copolymer could also be a multiblock copolymer having repeating BA or AB units of formula A(BA)n or B(AB)n, where n is an integer and wherein
A is selected from a group consisting of
   poly D-, L-, DL-lactic acid,
   poly D-, L-, DL-lactide,
   poly-glycolic acid,
   polyglycolide,
   polylactide-co-glycolide,
   poly-ε-caprolactone, and
   poly(3-hydroxybutyric acid); and
B is selected from a group of hydrophilic polymers consisting of
   polyvinylalcohol,
   polyvinylpyrrolidone,
   polyethylene oxide, and
   polyethylene glycol; or the hydrophilic polymer B may itself be a copolymer, for example a polyoxyethylone/polyoxypropylene block copolymer of the type known as Pluronics or synperonics.

WO99/18142 discloses certain pharmaceutical compositions which have in common the use of an aqueous triblock polymer solution that exhibits reverse gelation behaviour and a drug compound. WO00/19996 discloses certain pharmaceutical compositions that comprise retinoic acid incorporated into the microspheres prepared from di-block and tri-block copolymers and a biodegradable polymer. US5665428 discloses certain pharmaceutical compositions that comprise polypeptide or protein drug compounds and tri-block copolymers prepared as microspheres. EP1296647, published after the filing date of the present application, discloses certain pharmaceutical compositions that comprise micelle trapped drug compounds obtained by the formation of micelles poly(N-vinyl-2-pyrrolidone) copolymers in the presence of drug compounds.

Copolymers of the type described above are known, see for example US 4,942,035, USA 745,160, US4,526,938 or EP0,166,596,B1. Specifically these types of polymers are used in the formulation of parenteral compositions of drugs due to the ability of the copolymer to provide release of the drug over a prolonged period, several days. Previously it has not been thought that these polymers were suitable for oral administration due to the prolonged periods of release of drug, which would be unsuitable for achieving ideal oral adsorption of drug.

We have surprisingly found that such polymers are indeed suitable for oral administration of compounds and are particularly suitable for formulation to produce oral compositions of compounds with low aqueous solubility (less than 0.1mg/ml at the site of absorption). Whilst not wishing to be bound by theory we believe that these coploymers act by a combination of dissolution enhancement and prevention of precipitation and thus can greatly increase levels of drug absorption after oral administration.

In particular the polymers are particularly good with compounds which have significantly lower solubility in the pH conditions encountered at the site of adsorption, typically the duodenum, ileum or colon, than in the stomach. Typically these are basic compounds which are more soluble in the acidic stomach than the more alkaline conditions found in the site of absorption.

Compounds which have low aqueous solubility or basic compounds may produce problems in their absorption possibly producing unacceptable levels of variability in absorption between patient and between dose.

A common factor which may affect the absorption of a drug when administered orally is the changing pH experienced by the drug as it passes through the GI tract. Typically a drug may be absorbed in any number of the following sites when administered orally; cheek lining, stomach, duodenum, ileum and colon. The pH may be different at each site of adsorption with the pH significantly different from the stomach (pH 1-3.5) to the small intestine (pH 4-8). The solubility of the drug may vary with pH leading to the possibility of the drug coming out of solution as it passes through the GI tract. Particular difficulties exist where the drug is dissolved and the solubility decreases in the pH environment found at the site of adsorption. This leads to possible low absorption and variable adsorption between doses and different patients. For example we have found with the drug 1-(6-chloronaphth-2-ylsulfonyl)-4-[4-(4-pyridyl)benzoyl] piperazine (hereinafter referred to as Compound 1) is soluble within the acidic pH of the stomach, but is not adsorbed from this area, but has low solubility in the duodenum, ileum and colon which are the main sites of adsorption.

Compound 1 possesses Factor Xa inhibitory activity at concentrations which do not inhibit, or which inhibit to a lesser extent, the enzyme thrombin which is also a member of the blood coagulation enzymatic cascade.

Compound 1 is disclosed as Example 3 of WO9957113.

Compound 1 possesses activity in the treatment or prevention of a variety of medical disorders where anticoagulant therapy is indicated, for example in the treatment or prevention of thrombotic conditions such as coronary artery and cerebro-vascular disease. Further examples of such medical disorders include various cardiovascular and cerebrovascular conditions such as myocardial infarction, the formation of atherosclerotic plaques, venous or arterial thrombosis, coagulation syndromes, vascular injury (including reocclusion and restenosis following angioplasty and coronary artery bypass surgery, thrombus formation after the application of blood vessel operative techniques or after general surgery such as hip replacement surgery, the introduction of artificial heart valves or on the recirculation of blood), cerebral infarction, cerebral thrombosis, stroke, cerebral embolism, pulmonary embolism, ischaemia and angina (including unstable angina).

Standard tablet formulations of compound 1 may not be satisfactory due to the above reasons and have lead to poor oral bioavailability and most importantly high variability in adsorption. Variability is of most concern with any drug affecting the clotting cascade, care is needed since complete blockage of the clotting cascade is an unwanted side effect. On the other hand low exposure levels to the compound will not lead to any therapeutic benefit. Therefore, good oral bioavailability is required and, particularly, low variability.

We have found with the polymers described above that they act as solubilising enhancers as well as precipitation inhibitors, also the polymers are self dispersing, water miscible and micelle forming.

We present as a feature of the invention an oral pharmaceutical composition comprising a compound and a self dispersing, water miscible micelle forming diblock copolymer of formula AB or BA or a self dispersing, water miscible micelle forming triblock copolymer of formula ABA or BAB or a self dispersing, water miscible micelle forming multiblock copolymer having repeating BA or AB units of formula A(BA)n or B(AB)n, where n is an integer and wherein
A is selected from a group consisting of
   poly D-, L-, DL-lactic acid,
   poly D-, L-, DL-lactide,
   poly-glycolic acid,
   polyglycolide,
   polylactide-co-glycolide,
   poly-ε-caprolactone, and
   poly(3-hydroxybutyric acid); and
B is selected from a group of hydrophilic polymers consisting of
   polyvinylalcohol,
   polyvinylpyrrolidone,
   polyethylene oxide, and
   polyethylene glycol; or the hydrophilic polymer B may itself be a copolymer, for example a polyoxyethylene/polyoxypropylene block copolymer of the type known as Pluronics or synperonics,
   wherein the compound is a compound which has significantly lower solubility in the pH conditions encountered at the site of adsorption than in the stomach.
Ideally the copolymer is a diblock copolymer of formula AB or BA. However the copolymer could also be a triblock copolymer of formula ABA or BAB. The copolymer could also be a multiblock copolymer having repeating BA or AB units of formula A(BA)n or B(AB)n, where n is an integer (preferably the copolymer is a diblock copolymer of formula AB or BA) and wherein
A is selected from a group consisting of
   poly D-, L-, DL-lactic acid,
   poly D-, L-, DL-lactide,
   poly-glycolic acid,
   polyglycolide,
   polylactide-co-glycolide,
   poly-ε-caprolactone, and
   poly(3-hydroxybutyric acid); and
B is selected from a group of hydrophilic polymers consisting of
   polyvinylalcohol,
   polyvinylpyrrolidone,
   polyethylene oxide, and
   polyethylene glycol; or the hydrophilic polymer B may itself be a copolymer, for example a polyoxyethylene/polyoxypropylene block copolymer of the type known as Pluronics or synperonics.

A further feature of the invention is the use of a self dispersing, water miscible micelle forming diblock copolymer of formula AB or BA or a self dispersing, water miscible micelle forming triblock copolymer of formula ABA or BAB or a self dispersing, water miscible micelle forming multiblock copolymer having repeating BA or AB units of formula A(BA)n or B(AB)n, where n is an integer and wherein
A is selected from a group consisting of
   poly D-, L-, DL-lactic acid,
   poly D-, L-, DL-lactide,
   poly-glycolic acid,
   polyglycolide,
   polylactide-co-glycolide (PLGA),
   poly-ε-caprolactone, and
   poly(3-hydroxybutyric acid); and
B is selected from a group of hydrophilic polymers consisting of
   polyvinylalcohol,
   polyvinylpyrrolidone,
   polyethylene oxide, and
   polyethylene glycol; or the hy0.drophilic polymer B may itself be a copolymer, for example a polyoxyethylene/polyoxypropylene block copolymer of the type known as Pluronics or synperonics;
   in improving the oral bioavailabilty and/or variability of adsorption of a compound which has significantly lower solubility in the pH conditions encountered at the site of adsorption than in the stomach.
Ideally the copolymer is a diblock copolymer of formula AB or BA. However the copolymer could also be a triblock copolymer of formula ABA or BAB. The copolymer could also be a multiblock copolymer having repeating BA or AB units of formula A(BA)n or B(AB)n, where n is an integer (preferably the copolymer is a diblock copolymer of formula AB or BA) and wherein
A is selected from a group consisting of
   poly D-, L-, DL-lactic acid,
   poly D-, L-, Dr,-lactide,
   poly-glycolic acid,
   polyglycolide,
   polylactide-co-glycolide (PLGA),
   poly-ε-caprolactone, and
   poly(3-hydroxybutyric acid); and
B is selected from a group of hydrophilic polymers consisting of
   polyvinylalcohol,
   polyvinylpyrrolidone,
   polyethylene oxide, and
   polyethylene glycol; or the hydrophilic polymer B may itself be a copolymer, for example a polyoxyethylene/polyoxyprapylene block copolymer of the type known as Pluronics or synperonics;
   in improving the oral bioavailabilty and/or variability of adsorption of a compound.

The compound is an organic molecule of MW < 800, the formulation working best with compounds which are poorly aqueous soluble and also with a compound which is basic, adsorbed after administration in the small intestine and in which such compound has significantly lower solubility in the pH conditions found at the site of adsorption than in the stomach.

Preferably the copolymer is a diblock copolymer of formula AB or BA or triblock copolymer of formula ABA or BAB. More preferably the copolymer is a diblock copolymer of formula AB or BA. Preferably the A block segment of the block copolymer, is a poly-(D-,L- or DL-lactic acid) or poly (D-,L- or DL-lactide). Preferably the Mw is between 500 Da and 5000 Da. More preferably between 1000 Da and 3000 Da and even more preferably between 1500 Da and 2000 Da. Preferably the B block segment of the copolymer is a polyethylene glycol, preferably methoxy-polyethylene glycol. Preferably the Mw is between 500 Da and 10,000 Daltons, more preferably between 1,000 Da and 5000 Da.

The most preferred copolymer is an AB diblock copolymer where A is a poly-(D-,L-or DL-lactic acid) or poly (D-,L- or DL-lactide) of Mw 2000 Da and B is a methoxypolyethylene glycol of Mw 2000Da.

The polymer may be judged to be micelle forming by a person skilled in the art by determination of the Critical Micelle Concentration (cmc). The formation of micelles of the copolymer in an aqueous environment is supported by the detection of the cmc, which can be measured using the Wilhelmy plate method. (S.A Hagan, A.G.A Coombes, M.C. Garnett, S.E. Dunn, M.C. Davies, L. Illum and S.S. Davis, Langmuir 1996, 12, 2153-2161)

Methods for the preparation of the polymers used are described in US 4,942,035 and US4,526,938 or EP0,166,596,B1 Zhu. K.J, Lin. X.Z and Yang S.L. Preparation, characterisation and properties of polylactide (PLA)-poly(ethyleneglycol) (PEG) copolymers. J Appl. Polym. Sci., 39(1990)

By the use of the term "significantly lower solubility in the pH conditions found at the site of adsorption than in the stomach" we mean that the solubility of the compound is at least 10x more soluble in the pH conditions found in the stomach (pH1-2) than the pH conditions found in the small intestine, (pH6-9), preferably 20x, 30x, 40x, 50x and X100

We have found in *in vitro* tests that the maximum supersaturated concentration of Compound 1 is improved by 4-10 times by use of the polymers described above.

A preferred ratio of copolymer to compound is from 10:1 to 0.25:1. Preferably 5:1 to 1:1

A preferred compound is Compound 1, 1-(5-chloroindol-2-ylsulfonyl)-4-[4-(4-pyridyl)benzoyl] piperazine (hereinafter called Compound 2) and 1-(5-chloroindol-2-ylsulfonyl)-4-[4-(1-imidazolyl)benzoyl] piperazine (hereinafter called Compound 3). Compound 2 and Compound 3 are disclosed in Examples 3 and 6 respectively of WO9957113. Compound 2 and 3 like Compound 1 are Factor Xa inhibitors.

The composition may contain from 0.01mg to 1g of compound. Additional excipients may be included in the composition.

Typically the compound will be present in an amount within the range of 1 to 80%, and preferably from 1 to 50% (especially 2 to 15% 2 to 20%) by weight of the composition.

The composition may be made by admixture of the compound and polymer, preferably by cryo-grinding the polymer and mixing with the compound, compression then may be used. Preferred methods for preparing a composition is as a solid dispersion, such techniques are known in the art and typically comprise the steps of dissolving the compound and the polymer in a common solvent and evaporating the solvent. Methods for evaporating the solvent include rotary evaporation, spray drying with appropriate excipients, lyophilization and thin film evaporation. Other techniques may be used such as solvent controlled precipitation, pH controlled precipitation, supercritical fluid technology and hot melt extrusion. To aid the process the melt may be extruded with any necessary additional excipient such as a plasticiser, including supercritical fluids. With hot melt extrusion the melt may be extruded or filled directly into capsules

When referring to a solid dispersion we do not exclude the possibility that a proportion of the compound may be dissolved within the polymer used, the exact proportion, if any, will depend upon the physical properties of the compound and the polymer selected.

Conventional excipients which may be added include preservatives, stabilisers, antioxidants, silica flow conditioners, antiadherents or glidants.

The invention is illustrated below by the following non-limiting examples.

### Preparation of solid dispersion

### For a 1:5 ratio

0.5g of drug (Compound 1) and 2.5g of polymer are weighed directly into a 250ml round bottom flask and dissolved in 63ml of methanol/dichloromethane (50:50). The solvent was removed on the rotary evaporator. The formulation was placed in a vacuum oven and dried under high vacuum at 40°C for 48hours.
Weights and volumes for other ratio's are pro-rata to the above formulation.

**Solubility Measurements**

| Solubility | Compound 1 |
|---|---|
| Water | <5ug/ml |
| pH1.2 | 250ug/ml |
| pH6.8 | 2ug/ml |

### In vitro dissolution of solid dispersions

### pH shift dissolution method

The formulations were weighed into hard gelatin capsules (equivalent to 25mg drug) and dissoluted in 500ml 0.1N HCl for one hour at 37°C (paddle speed 100rpm). A 5ml sample was taken at 55minutes and the media replaced. After one hour 10ml of a 2.5M KH₂PO₄ / 16.72% (w/v) NaOH solution was added to the HCl to shift the pH to 6.5. 5ml samples were then removed with a plastic syringe at 5, 15, 30, 45 and 60 minutes and media replaced after every sampling time point. Each sample was centrifuged (14,000rpm) at ambient temperature for 15 minutes and then analysed by HPLC using the following conditions:

| | |
|---|---|
| Eluent: | 40% ACN / 60% water / 0.2% TFA |
| column: | 25cm HIRPB 4.6mm i.d.. (with guard) |
| detection wavelength: | 236nm |
| flow rate: | 1.5ml/min |
| temperature: | ambient |
| injection volume: | 80µl |
| retention time: | approximately 6 minutes |

### pH 6.5 dissolution method

The formulations were weighed into hard gelatin capsules (equivalent to 25mg drug) and dissoluted in media comprising of 500ml 0.1N HCl and 10ml of a 2.5M KH₂PO₄ / 16.72% (w/v) NaOH solution for one hour at 37°C (paddle speed 100rpm). 5ml samples were then removed with a plastic syringe at 5, 10, 20, 30, 45 and 60 minutes and media replaced after every sampling time point. Each sample was centrifuged (14,000rpm) at ambient temperature for 15 minutes and then analysed by HPLC using the same conditions as the pH shift method.

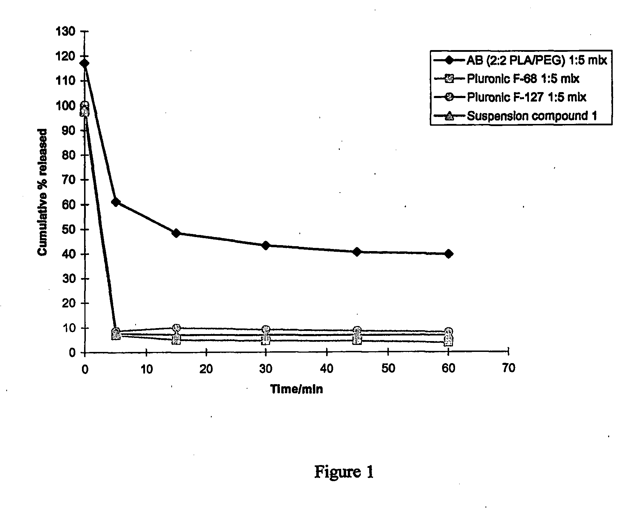

Figure 1 shows the release profile of a solid dispersion of Compound 1 with a PLA:PEG AB block copolymer and Pluronic polymers using the pH shift dissolution method. A conventional suspension of Compound 1 was included for comparison. This figure demonstrates that the PLA:PEG polymer is the optimal solid dispersion matrix material since the highest levels of supersaturation are attained with this polymer. The solid dispersions made with Pluronic F-68 and F-127 do not provide any great advantage over a conventional suspension of Compound 1. Similarly to the conventional suspension, on shifting to the higher pH, the Pluronic formulations are not capable of maintaining supersaturated levels.

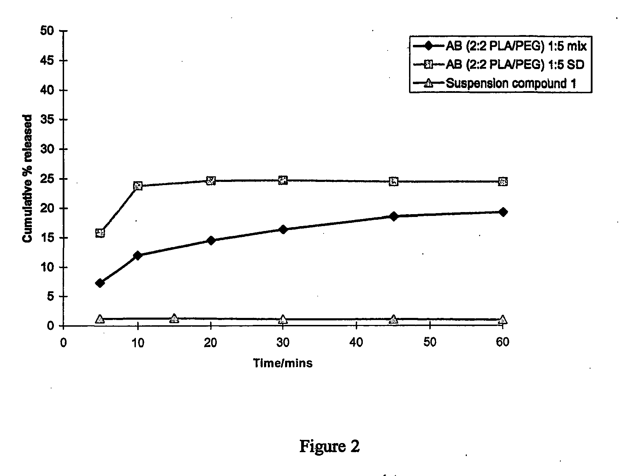

Figure 2 shows the release profile of two PLA:PEG AB block copolymer formulations of Compound 1 (SD is a solid dispersion and mix is an admixture) in the pH 6.5 dissolution test. A conventional suspension of Compound 1 was included for comparison. This figure demonstrates that in the absence of any prior formulation, the PLA:PEG polymer is capable of enhancing the dissolution of Compound 1 (admixture). This may be as a result of the polymer solubilising the compound.

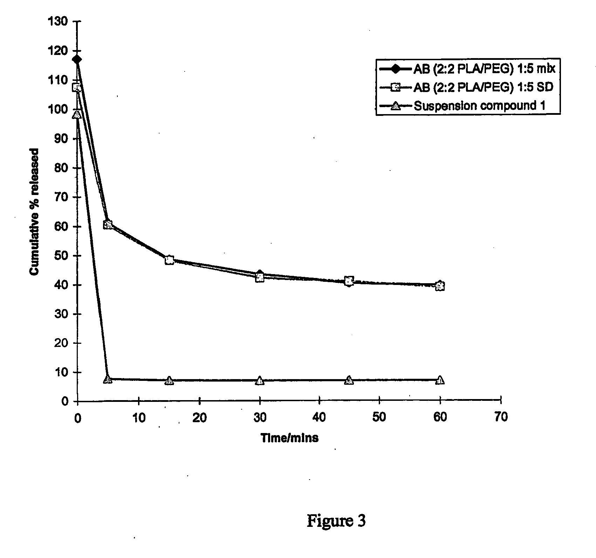

Figure 3 shows the release profile of two PLA:PEG AB block copolymer formulations of Compound 1 (SD is a solid dispersion and mix is an admixture) in the pH shift dissolution test. A conventional suspension of Compound 1 was included for comparison. This figure demonstrates that the PLA:PEG polymer is capable of maintaining supersaturated levels of the compound 1 in both the formulated and non-formulated state (i.e. SD or mix). Figures 2 and 3 demonstrate that the PLA:PEG's could be acting by a combination of solubilisation and inhibition of precipitation.

## Claims

1. An oral pharmaceutical composition comprising a compound and a self dispersing, water miscible micelle forming diblock copolymer of formula AB or BA or a self dispersing, water miscible micelle forming triblock copolymer of formula ABA or BAB or a self dispersing, water miscible micelle forming multiblock copolymer having repeating BA or AB units of formula A(BA)n or B(AB)n, where n is an integer and wherein
A is selected from a group consisting of
poly D-, L-, DL-lactic acid,
poly D-, L-, DL-lactide,
poly-glycolic acid,
polyglycolide,
polylactide-co-glycolide,
poly-ε-caprolactone, and
poly(3-hydroxybutyric acid); and
B is selected from a group of hydrophilic polymers consisting of
polyvinylalcohol,
polyvinylpyrrolidone,
polyethylene oxide, and
polyethylene glycol; or the hydrophilic polymer B may itself be a copolymer, for example a polyoxyethylene/polyoxypropylene block copolymer of the type known as Pluronics or synperonics,
wherein the compound is a compound which has significantly lower solubility in the pH conditions encountered at the site of adsorption than in the stomach.

2. Use of a self dispersing, water miscible micelle forming diblock copolymer of formula AB or BA or a self dispersing, water miscible micelle forming triblock copolymer of formula ABA or BAB or a self dispersing, water miscible micelle forming multiblock copolymer having repeating BA or AB units of formula A(BA)n or B(AB)n, where n is an integer and
wherein
A is selected from a group consisting of
poly D-, L-, DL-lactic acid,
poly D-, L-, DL-lactide,
poly-glycolic acid,
polyglycolide,
polylactide-co-glycolide (PLGA),
poly-ε-caprolactone, and
poly(3-hydroxybutyric acid); and
B is selected from a group of hydrophilic polymers consisting of
polyvinylalcohol,
polyvinylpyrrolidone,
polyethylene oxide, and
polyethylene glycol; or the hydrophilic polymer B may itself be a copolymer, for example a polyoxyethylene/polyoxypropylene block copolymer of the type known as Pluronics or synperonics;
in improving the oral bioavailabilty and/or variability of adsorption of a compound which has significantly lower solubility in the pH conditions encountered at the site of adsorption than in the stomach.

3. An oral pharmaceutical composition as claimed in claim 1 or use of a self dispersing, water miscible micelle forming copolymer as claimed in claim 2 wherein the compound is basic.

4. An oral pharmaceutical composition as claimed in claim 1 or 3 or use of a self dispersing, water miscible micelle forming copolymer as claimed in claim 2 or 3 wherein the compound is an organic molecule of MW<800.

5. An oral pharmaceutical composition as claimed in claim 1, 3 or 4 wherein the composition is obtainable by admixing the compound and the polymer; dissolving the compound and the polymer in a common solvent and evaporating the solvent; solvent controlled precipitation; pH controlled precipitation; supercritical fluid technology; or hot melt extrusion.

6. The use of a self dispersing, water miscible micelle forming copolymer as claimed in claim 2, 3 or 4 wherein an oral pharmaceutical composition, comprising the compound and the polymer, obtainable by admixing the compound and the polymer; dissolving the compound and the polymer in a common solvent and evaporating the solvent; solvent controlled precipitation; pH controlled precipitation; supercritical fluid technology, or hot melt extrusion is used.

7. An oral pharmaceutical composition as claimed in claim 1, 3, 4 or 5 or use of a self dispersing, water miscible micelle forming copolymer as claimed in claim 2, 3, 4 or 6 wherein the A block segment of the copolymer is a poly-(D-, L- or DL-lactic acid) or poly (D-, L- or DL-lactide).

8. An oral pharmaceutical composition as claimed in claim 7 or use of a self dispersing, water miscible micelle forming copolymer as claimed in claim 7 wherein the Mw of the A polymer is between 500 Da and 5,000 Da.

9. An oral pharmaceutical composition as claimed in claim 8 or use of a self dispersing, water miscible micelle forming copolymer as claimed in claim 8 wherein the Mw of the A polymer is between 1000 Da and 3000 Da.

10. An oral pharmaceutical composition as claimed in claim 9 or use of a self dispersing, water miscible micelle forming copolymer as claimed in claim 9 wherein the Mw of the A polymer is between 1300 Da and 2200 Da.

11. An oral pharmaceutical composition as claimed in claim 10 or use of a self dispersing, water miscible micelle forming copolymer as claimed in claim 10 wherein the Mw of the A polymer is 2000 Da.

12. An oral pharmaceutical composition as claimed in any one of claims 1, 3, 4, 5, 7, 8, 9, 10 or 11 or use of a self dispersing, water miscible micelle forming copolymer as claimed in any one of claims 2, 3, 4, 6, 7, 8, 9, 10 or 11 wherein the B block segment of the copolymer is a polyethylene glycol.

13. An oral pharmaceutical composition as claimed in claim 12 or use of a self dispersing, water miscible micelle forming copolymer as claimed in claim 12 wherein the B block segment of the copolymer is methoxy-polyethylene glycol.

14. An oral pharmaceutical composition as claimed in claim 12 or 13 or use of a self dispersing, water miscible micelle forming copolymer as claimed in claim 12 or 13 wherein the Mw of the B polymer is between 500 Da and 10,000 Da.

15. An oral pharmaceutical composition as claimed in claim 14 or use of a self dispersing, water miscible micelle forming copolymer as claimed in claim 14 wherein the Mw of the B polymer is between 1,000 Da and 5000 Da.

16. An oral pharmaceutical composition as claimed in any one of claims 1, 3, 4, 5 or 7 to 15 or use of a self dispersing, water miscible micelle forming copolymer as claimed in any one of claims 2, 3, 4, or 6 to 15 wherein the copolymer is a diblock copolymer of formula AB or BA.

17. An oral pharmaceutical composition as claimed in any one of claims 1, 3, 4, 5 or 7 to 15 or use of a self dispersing, water miscible micelle forming copolymer as claimed in any one of claims 2 3, 4, or 6 to 15 wherein the copolymer is a triblock copolymer of formula ABA or BAB.

18. An oral pharmaceutical composition as claimed in Claim 16 comprising a compound and a diblock copolymer of formula AB or BA wherein A is a polyL-lactide of Mw of 2000 Da and B is a polyethylene glycol of Mw of 2000 Da.

19. An oral pharmaceutical composition as claimed in Claim 16 comprising a compound and a diblock copolymer of formula AB or BA wherein A is a poly-(D-, L- or DL-lactic acid) or poly (D-, L- or DL-lactide) of Mw 2000 Da and B is a methoxypolyethylene glycol of Mw 2000Da.

20. An oral pharmaceutical composition as claimed in any one of claims 1, 3, 4, 5, 7 to 19 wherein the compound is selected from 1-(6-chloronaphth-2-ylsulfonyl)-4-[4-(4-pyridyl)benzoyl] piperazine, 1-(5-chloroindol-2-ylsulfonyl)-4-[4-(4-pyridyl)benzoyl] piperazine and 1-(5-chloroindol-2-ylsulfonyl)-4-[4-(1-imidazolyl)benzoyl] piperazine.

21. An oral pharmaceutical composition as claimed in any one of claims 1, 3, 4, 5, 7 to 20 wherein the ratio of copolymer to compound is from 10:1 1 to 0.25:1.

22. An oral pharmaceutical composition as claimed in any one of claims 1, 3, 4, 5, 7 to 21 wherein the composition comprises from 0.01 mg to 1 mg of compound.

## Patentansprüche

1. Orale pharmazeutische Zusammensetzung, enthaltend eine Verbindung und ein selbstdispergierendes, wassermischbares micellenbildendes Diblockcopolymer der Formel AB oder BA oder ein selbstdispergierendes, wassermischbares micellenbildendes Triblockcopolymer der Formel ABA oder BAB oder ein selbstdispergierendes, wassermischbares micellenbildendes Multiblockcopolymer mit sich wiederholenden BA- oder AB-Einheiten der Formel A(BA)ₙ oder B(AB)ₙ, worin n für eine ganze Zahl steht und
A ausgewählt ist aus der Gruppe bestehend aus
Poly-D-, -L-, -DL-milchsäure,
Poly-D-, -L-, -DL-lactid,
Polyglykolsäure,
Polyglykolid,
Polylactid-co-glycolid,
Poly-ε-caprolacton und
Poly(3-hydroxybuttersäure); und
B ausgewählt ist aus der Gruppe von hydrophilen Polymeren bestehend aus
Polyvinylalkohol,
Polyvinylpyrrolidon,
Polyethylenoxid und
Polyethylenglykol; oder es sich bei dem hydrophilen Polymer B selbst um ein Copolymer handeln kann, beispielsweise ein Polyoxyethylen/Polyoxypropylen-Blockcopolymer des unter der Bezeichnung Pluronics oder Synperonics bekannten Typs,
wobei es sich bei der Verbindung um eine Verbindung handelt, die bei den an der Resorptionsstelle anzutreffenden pH-Bedingungen eine erheblich geringere Löslichkeit aufweist als im Magen.

2. Verwendung eines selbstdispergierenden, wassermischbaren micellenbildenden Diblockcopolymers der Formel AB oder BA oder eines selbstdispergierenden, wassermischbaren micellenbildenden Triblockcopolymers der Formel ABA oder BAB oder eines selbstdispergierenden, wassermischbaren micellenbildenden Multiblockcopolymers mit sich wiederholenden BA- oder AB-Einheiten der Formel A(BA)ₙ oder B(AB)ₙ, worin n für eine ganze Zahl steht und
A ausgewählt ist aus der Gruppe bestehend aus
Poly-D-, -L-, -DL-milchsäure,
Poly-D-, -L-, -DL-lactid,
Polyglykolsäure,
Polyglykolid,
Polylactid-co-glycolid,
Poly-ε-caprolacton und
Poly(3-hydroxybuttersäure); und
B ausgewählt ist aus der Gruppe von hydrophilen Polymeren bestehend aus
Polyvinylalkohol,
Polyvinylpyrrolidon,
Polyethylenoxid und
Polyethylenglykol; oder es sich bei dem hydrophilen Polymer B selbst um ein Copolymer handeln kann, beispielsweise ein Polyoxyethylen/Polyoxypropylen-Blockcopolymer des unter der Bezeichnung Pluronics oder Synperonics bekannten Typs,
zur Verbesserung der oralen Bioverfügbarkeit und/oder Variabilität der Resorption einer Verbindung, die bei den an der Resorptionsstelle anzutreffenden pH-Bedingungen eine erheblich geringere Löslichkeit aufweist als im Magen.

3. Orale pharmazeutische Zusammensetzung nach Anspruch 1 oder Verwendung eines selbstdispergierenden, wassermischbaren micellenbildenden Copolymers nach Anspruch 2, wobei die Verbindung basisch ist.

4. Orale pharmazeutische Zusammensetzung nach Anspruch 1 oder 3 oder Verwendung eines selbstdispergierenden, wassermischbaren micellenbildenden Copolymers nach Anspruch 2 oder 3, wobei es sich bei der Verbindung um ein organisches Molekül mit Mw < 800 handelt.

5. Orale pharmazeutische Zusammensetzung nach Anspruch 1, 3 oder 4, die durch Mischen der Verbindung und des Polymers, Lösen der Verbindung und des Polymers in einem gemeinsamen Lösungsmittel und Verdampfen des Lösungsmittels; lösungsmittelkontrollierte Fällung; pH-kontrollierte Fällung; Techniken unter Verwendung von überkritischen Fluiden oder Extrusion nach dem Heißschmelzverfahren erhältlich ist.

6. Verwendung eines selbstdispergierenden, wassermischbaren micellenbildenden Copolymers nach Anspruch 2, 3 oder 4, wobei eine orale pharmazeutische Zusammensetzung verwendet wird, die die Verbindung und das Polymer enthält und durch Mischen der Verbindung und des Polymers, Lösen der Verbindung und des Polymers in einem gemeinsamen Lösungsmittel und Verdampfen des Lösungsmittels; lösungsmittelkontrollierte Fällung; pH-kontrollierte Fällung; Techniken unter Verwendung von überkritischen Fluiden oder Extrusion nach dem Heißschmelzverfahren erhältlich ist.

7. Orale pharmazeutische Zusammensetzung nach Anspruch 1, 3, 4 oder 5 oder Verwendung eines selbstdispergierenden, wassermischbaren micellenbildenden Copolymers nach Anspruch 2, 3, 4 oder 6, wobei es sich bei dem A-Block-Segment des Copolymers um Poly(D- L- oder DL-milchsäure) oder Poly(D-, L- oder DL-lactid) handelt.

8. Orale pharmazeutische Zusammensetzung nach Anspruch 7 oder Verwendung eines selbstdispergierenden, wassermischbaren micellenbildenden Copolymers nach Anspruch 7, wobei das Mw des Polymers A zwischen 500 Da und 5000 Da liegt.

9. Orale pharmazeutische Zusammensetzung nach Anspruch 8 oder Verwendung eines selbstdispergierenden, wassermischbaren micellenbildenden Copolymers nach Anspruch 8, wobei das Mw des Polymers A zwischen 1000 Da und 3000 Da liegt.

10. Orale pharmazeutische Zusammensetzung nach Anspruch 9 oder Verwendung eines selbstdispergierenden, wassermischbaren micellenbildenden Copolymers nach Anspruch 9, wobei das Mw des Polymers A zwischen 1300 Da und 2200 Da liegt.

11. Orale pharmazeutische Zusammensetzung nach Anspruch 10 oder Verwendung eines selbstdispergierenden, wassermischbaren micellenbildenden Copolymers nach Anspruch 10, wobei das Mw des Polymers A 2000 Da beträgt.

12. Orale pharmazeutische Zusammensetzung nach einem der Ansprüche 1, 3, 4, 5, 7, 8, 9, 10 oder 11 oder Verwendung eines selbstdispergierenden, wassermischbaren micellenbildenden Copolymers nach einem der Ansprüche 2, 3, 4, 6, 7, 8, 9, 10 oder 11, wobei es sich bei dem B-Block-Segment des Copolymers um ein Polyethylenglykol handelt.

13. Orale pharmazeutische Zusammensetzung nach Anspruch 12 oder Verwendung eines selbstdispergierenden, wassermischbaren micellenbildenden Copolymers nach Anspruch 12, wobei es sich bei dem B-Block-Segment des Copolymers um Methoxypolyethylenglykol handelt.

14. Orale pharmazeutische Zusammensetzung nach Anspruch 12 oder 13 oder Verwendung eines selbstdispergierenden, wassermischbaren micellenbildenden Copolymers nach Anspruch 12 oder 13, wobei das Mw des Polymers B zwischen 500 Da und 10.000 Da liegt.

15. Orale pharmazeutische Zusammensetzung nach Anspruch 14 oder Verwendung eines selbstdispergierenden, wassermischbaren micellenbildenden Copolymers nach Anspruch 14, wobei das Mw des Polymers B zwischen 1000 Da und 5000 Da liegt.

16. Orale pharmazeutische Zusammensetzung nach einem der Ansprüche 1, 3, 4, 5 oder 7 bis 15 oder Verwendung eines selbstdispergierenden, wassermischbaren micellenbildenden Copolymers nach einem der Ansprüche 2, 3, 4 oder 6 bis 15, wobei es sich bei dem Copolymer um ein Diblockcopolymer der Formel AB oder BA handelt.

17. Orale pharmazeutische Zusammensetzung nach einem der Ansprüche 1, 3, 4, 5 oder 7 bis 15 oder Verwendung eines selbstdispergierenden, wassermischbaren micellenbildenden Copolymers nach einem der Ansprüche 2, 3, 4 oder 6 bis 15, wobei es sich bei dem Copolymer um ein Triblockcopolymer der Formel ABA oder BAB handelt.

18. Orale pharmazeutische Zusammensetzung nach Anspruch 16, enthaltend eine Verbindung und ein Diblockcopolymer der Formel AB oder BA, worin A für ein Poly-L-lactid mit einem Mw von 2000 Da steht und B für ein Polyethylengylkol mit einem Mw von 2000 Da steht.

19. Orale pharmazeutische Zusammensetzung nach Anspruch 16, enthaltend eine Verbindung und ein Diblockcopolymer der Formel AB oder BA, worin A für eine Poly(D-, L- oder DL-milchsäure) oder ein Poly(D-, L- oder DL-lactid) mit einem Mw von 2000 Da steht und B für ein Methoxypolyethylengylkol mit einem Mw von 2000 Da steht.

20. Orale pharmazeutische Zusammensetzung nach einem der Ansprüche 1, 3, 4, 5 oder 7 bis 19, wobei die Verbindung unter 1-(6-Chlornaphth-2-ylsulfonyl)-4-[4-(4-pyridyl)benzoyl]piperazin, 1-(5-Chlorindol-2-ylsulfonyl) -4- [4- (4-pyridyl) benzoyl] piperazin und 1-(5-Chlorindol-2-ylsulfonyl)-4-[4-(1-imidazolyl)benzoyl]piperazin ausgewählt ist.

21. Orale pharmazeutische Zusammensetzung nach einem der Ansprüche 1, 3, 4, 5 oder 7 bis 20, wobei das Verhältnis von Copolymer zu Verbindung 10:1 bis 0,25:1 beträgt.

22. Orale pharmazeutische Zusammensetzung nach einem der Ansprüche 1, 3, 4, 5 oder 7 bis 21, wobei die Zusammensetzung 0,01 mg bis 1 mg Verbindung enthält.

## Revendications

1. Composition pharmaceutique orale comprenant un composé et un copolymère dibloc micellisable miscible à l'eau, autodispersant de formule AB ou BA ou un copolymère tribloc micellisable miscible à l'eau, autodispersant de formule ABA ou BAB ou un copolymère multibloc micellisable miscible à l'eau, autodispersant ayant des motifs répétitifs BA ou AB de formule A(BA)n ou B(AB)n, où n est un entier et où
A est choisi dans le groupe constitué de
poly(acide) D-, L-, DL-lactique,
poly(D-, L-, DL-lactide),
poly(acide) glycolique,
polyglycolide,
poly(lactide-co-glyCOlide),
poly-ε-caprolactone, et
poly(acide 3-hydroxybutyrique) ; et
B est choisi dans le groupe de polymères hydrophiles constitués de
alcool polyvinylique,
polyvinylpyrrolidone,
paly(oxyde d'éthylène), et
polyéthylèneglycol ; ou le polymère hydrophile B peut lui-même être un copolymère, par exemple un copolymère bloc polyoxyéthylène/polyoxypropylène de type connu tel que les Pluronics ou les Synperonics,
où le composé est un composé qui a une solubilité significativement plus basse dans les conditions de pH rencontrées au site d'adsorption que dans l'estomac.

2. Utilisation d'un copolymère dibloc micellisable miscible à l'eau, autodispersant de formule AB ou BA ou d'un copolymère tribloc micellisable miscible à l'eau, autodispersant de formule ABA ou BAB ou d'un copolymère multibloc micellisable miscible à l'eau, autodispersant ayant les motifs répétitifs BA ou AB de formule A (BA) n ou B(AB)n, où n est un entier et où
A est choisi dans le groupe constitué de
poly(acide) D-, L-, DL-lactique,
poly (D-, L-, DL-lactide),
poly(acide) glycolique,
polyglycolide,
poly(lactide-co-glycolide), (PLGA)
poly-ε-caprolactone, et
poly(acide 3-hydroxybutyrique) ; et
B est choisi dans le groupe de polymères hydrophiles constitués de
alcool polyvinylique,
polyvinylpyrrolidone,
poly(oxyde d'éthylène), et
polyéthylèneglycol ; ou le polymère hydrophile B peut lui-même être un copolymère, par exemple un copolymère bloc polyoxyéthylène/polyoxypropylène de type connu tel que les Pluronics ou les Synperonics ;
pour améliorer la biodisponibilité par voie orale et/ou la variabilité d'adsorption d'un composé qui a une solubilité significativement plus basse dans les conditions de pH rencontrées au site d'adsorption que dans l'estomac.

3. Composition pharmaceutique orale selon la revendication 1, ou utilisation d'un copolymère micellisable miscible à l'eau, autodispersant selon la revendication 2, où le composé est basique.

4. Composition pharmaceutique orale selon la revendication 1 ou 3, ou utilisation d'un copolymère micellisable miscible à l'eau, autodispersant selon la revendication 2 ou 3, où le composé est une molécule organique de PM < 800.

5. Composition pharmaceutique orale selon la revendication 1, 3 ou 4, **caractérisée en ce que** la composition est susceptible d'être obtenue par mélange du composé et du polymère ; dissolution du composé et du polymère dans un solvant courant et évaporation du solvant ; précipitation contrôlée par le solvant ; précipitation contrôlée par le pH ; technologie de fluides supercritiques ; ou extrusion en fusion.

6. Utilisation d'un copolymère micellisable miscible à l'eau, autodispersant selon la revendication 2, 3 ou 4, **caractérisée en ce qu'**on utilise une composition pharmaceutique orale, comprenant le composé et le polymère, susceptible d'être obtenue par mélange du composé et du polymère ; dissolution du composé et du polymère dans un solvant courant et évaporation du solvant ; précipitation contrôlée par le solvant ; précipitation contrôlée par le pH ; technologie de fluides supercritiques ; ou extrusion en fusion.

7. Composition pharmaceutique orale selon la revendication 1, 3, 4 ou 5 ou utilisation d'un copolymère micellisable miscible à l'eau, autodispersant selon la revendication 2, 3, 4 ou 6, où le segment de bloc A du copolymère est un poly(acide D-, L- ou DL-lactique) ou poly(D-, L-ou DL-lactide).

8. Composition pharmaceutique orale selon la revendication 7 ou utilisation d'un copolymère micellisable miscible à l'eau, autodispersant selon la revendication 7, où le Mw du polymère A est compris entre 500 Da et 5000 Da.

9. Composition pharmaceutique orale selon la revendication 8 ou utilisation d'un copolymère micellisable miscible à l'eau, autodispersant selon la revendication 8, où le Mw du polymère A est compris entre 1000 Da et 3000 Da.

10. Composition pharmaceutique orale selon la revendication 9 ou utilisation d'un copolymère micellisable miscible à l'eau, autodispersant selon la revendication 9, où le Mw du polymère A est compris entre 1300 Da et 2200 Da.

11. Composition pharmaceutique orale selon la revendication 10 ou utilisation d'un copolymère micellisable miscible à l'eau, autodispersant selon la revendication 10, où le Mw du polymère A est 2000 Da.

12. Composition pharmaceutique orale selon l'une quelconque des revendications 1, 3, 4, 5, 7, 8, 9, 10 ou 11 ou utilisation d'un copolymère micellisable miscible à l'eau, autodispersant selon l'une quelconque des revendications 2, 3, 4, 6, 7, 8, 9, 10 ou 11 où le segment de bloc B du copolymère est un polyéthylèneglycol.

13. Composition pharmaceutique orale selon la revendication 12 ou utilisation d'un copolymère micellisable miscible à l'eau, autodispersant selon la revendication 12, où le segment de bloc B du copolymère est méthoxy-polyéthylèneglycol.

14. Composition pharmaceutique orale selon la revendication 12 ou 13 ou utilisation d'un copolymère micellisable miscible à l'eau, autodispersant selon la revendication 12 ou 13, où le Mw du polymère B est compris entre 500 Da et 10 000 Da.

15. Composition pharmaceutique orale selon la revendication 14 ou utilisation d'un copolymère micellisable miscible à l'eau, autodispersant selon la revendication 14, où le Mw du polymère B est compris entre 1000 Da et 5000 Da.

16. Composition pharmaceutique orale selon l'une quelconque des revendications 1, 3, 4, 5 ou 7 à 15 ou utilisation d'un copolymère micellisable miscible à l'eau, autodispersant selon l'une quelconque des revendications 2, 3, 4 ou 6 à 15, où le copolymère est un copolymère dibloc de formule AB ou BA.

17. Composition pharmaceutique orale selon l'une quelconque des revendications 1, 3, 4, 5 ou 7 à 15 ou utilisation d'un copolymère micellisable miscible à l'eau, autodispersant selon l'une quelconque des revendications 2, 3, 4 ou 6 à 15, où le copolymère est un copolymère tribloc de formule ABA ou BAB.

18. Composition pharmaceutique orale selon la revendication 16, comprenant un composé et un copolymère dibloc de formule AB ou BA, où A est un poly(L-lactide) de Mw de 2000 Da et B est un polyéthylèneglycol de Mw de 2000 Da.

19. Composition pharmaceutique orale selon la revendication 16, comprenant un composé et un copolymère dibloc de formule AB ou BA où A est un poly(acide D-, L- ou DL-lactique) ou poly(D-, L-ou DL-lactide) de Mw de 2000 Da et B est un méthoxypolyéthylèneglycol de Mw de 2000 Da.

20. Composition pharmaceutique orale selon l'une quelconque des revendications 1, 3, 4, 5, 7 à 19, **caractérisée en ce que** le composé est choisi parmi la 1-(6-chloronapht-2-ylsulfonyl)-4-[4-(4-pyridyl)benzoyl]pipérazine, la 1-(5-chloroindol-2-ylsulfonyl)-4-[4-(4-pyridyl)benzoyl]pipérazine et la 1-(5-chloroindol-2-ylsulfonyl)-4-[4-(1-imidazolyl)benzoyl]pipérazine.

21. Composition pharmaceutique orale selon l'une quelconque des revendications 1, 3, 4, 5, 7 à 20, **caractérisée en ce que** le rapport du copolymère au composé est de 10:1 à 0,25:1.

22. Composition pharmaceutique orale selon l'une quelconque des revendications 1, 3, 4, 5, 7 à 21, **caractérisée en ce que** la composition comprend de 0,01 mg à 1 mg de composé.
